Europäisches Patentamt

European Patent Office

Office européen des brevets

(11) Veröffentlichungsnummer: **0 318 924 B1**

(12) **EUROPÄISCHE PATENTSCHRIFT**

(45) Veröffentlichungstag der Patentschrift: **18.03.92**

(21) Anmeldenummer: **88119889.9**

(22) Anmeldetag: **29.11.88**

(51) Int. Cl.5: **A61M 5/14**

(54) **Auftau- und Temperiervorrichtung für medizinische Produkte.**

(30) Priorität: **04.12.87 DE 3741051**

(43) Veröffentlichungstag der Anmeldung:
**07.06.89 Patentblatt 89/23**

(45) Bekanntmachung des Hinweises auf die
Patenterteilung:
**18.03.92 Patentblatt 92/12**

(84) Benannte Vertragsstaaten:
**AT BE CH DE FR GB LI LU NL**

(56) Entgegenhaltungen:
**DE-U- 8 606 622**
**US-A- 3 518 393**
**US-A- 4 473 739**

(73) Patentinhaber: **Barkey, Volker**
**Herforder Strasse 176**
**W-4800 Bielefeld 1(DE)**

(72) Erfinder: **Barkey, Volker**
**Herforder Strasse 176**
**W-4800 Bielefeld 1(DE)**

(74) Vertreter: **TER MEER - MÜLLER - STEINMEI-**
**STER & PARTNER**
**Artur-Ladebeck-Strasse 51**
**W-4800 Bielefeld 1(DE)**

## Beschreibung

Die Erfindung betrifft eine Auftau- und Temperiervorrichtung für medizinische Produkte, insbesondere für tiefgefrorene Plasma- oder Blutkonserven, mit wenigstens zwei ein temperiertes Medium enthaltenden, beidseitig eines zu temperierenden Produkts anzuordnenden Kunststoffbeuteln.

Eine derartige Vorrichtung ist aus dem DE-GM 86 06 622 des Anmelders bekannt. Zahlreiche medizinische Produkte, insbesondere Plasmalösungen oder Blutkonserven, werden bei minus 20°C in Tiefkühlschränken oder -truhen gelagert und müssen bei Bedarf auf der Krankenstation innerhalb kurzer Zeit auf z.B. 37°C erwärmt werden. Dabei ist eine örtliche Überhitzung der Konserve zu vermeiden, da sie etwa bei Blutkonserven zu einer Gerinnung oder bei anderen Konserven zu unerwünschten chemischen Reaktionen führen kann.

In der Praxis weit verbreitet ist die Verwendung eines Wasserbades zum Auftauen von tiefgefrorenen Produkten, jedoch ermöglicht warmes Wasser von ca. 37°C eine geradezu sprunghafte Vermehrung von Bakterien, Viren und Pilzen, die auf den Plasmabeutel übertragen und auch anderweitig verschleppt werden. Demgegenüber wird durch das obige Gebrauchsmuster der Vorteil erreicht, daß die Konserve trokken, also ohne Berührung mit einem Wasserbad temperiert werden kann, so daß die Gefahr der Ausbreitung von Bakterien, Viren und Pilzen stark eingeschränkt wird. Trotzdem ermöglicht die Verwendung von zwei mit warmem Wasser oder einem anderen Temperiermedium gefüllten Beutel, die die Konserve großflächig und eng umschließen, einen ähnlich raschen Wärmeübergang, wie es bei Wasserbädern der Fall ist. Die erreichbaren Temperierzeiten sind also verhältnismäßig kurz, wenngleich eine weitere Verkürzung naturgemäß wünschenswert wäre.

Der Erfindung liegt die Aufgabe zugrunde, eine Vorrichtung zu schaffen, die es ermöglicht, die Temperiergeschwindigkeit gegenüber der bekannten Vorrichtung weiter zu steigern, ohne daß die Gefahr einer Überhitzung und Beschädigung des Produkts besteht.

Diese Aufgabe wird erfindungsgemäß bei einer Vorrichtung der eingangs genannten Art gelöst durch wenigstens ein periodisch auf wenigstens einen der Kunststoffbeutel einwirkendes Druckglied.

Durch das Druckglied wird das Temperiermedium in dem Kunststoffbeutel in Schwingungen versetzt, so daß eine Schaukelbewegung entsteht, die sich dem medizinischen Produkt, beispielsweise einem Plasmabeutel mitteilt. Dadurch gerät auch das Produkt in Bewegung, so daß der Temperaturausgleich von außen nach innen und über das gesamte Produkt beschleunigt wird. Es wird daher eine gleichmäßige und rasche Bewegung ohne örtliche Überhitzungen erreicht. Unter Umständen besteht sogar die Möglichkeit, die Temperatur des Temperiermediums gegenüber der angestrebten Endtemperatur des Produkts in gewissen Grenzen zu erhöhen und damit eine weitere Beschleunigung zu erzielen, da durch die Schaukelbewegung und den raschen Temperaturausgleich örtliche Überhitzungen an der Außenfläche des Produkts vermieden werden können.

Vorzugsweise weist die Vorrichtung zwei aufeinander liegende, im wesentlichen deckungsgleiche, mit dem temperierten Medium gefüllte Kunststoffbeutel auf, zwischen die das zu temperierende Produkt nach Abheben des oberen Beutels gelegt wird, und das Druckglied besteht aus einem zwischen die Kunststoffbeutel eintretenden, drehbaren Exzenter, der abwechselnd auf die Kunststoffbeutel einwirkt.

Bei einer Vorrichtung der angegebenen Art, bei der die Kunststoffbeutel ein mit Hilfe einer Pumpe umgewälztes, temperiertes Medium enthalten, wird vorzugsweise der Innendruck bzw. Füllungsgrad der Kunststoffbeutel während eines Schaukelvorganges abgesenkt. Auf diese Weise läßt sich das temperierte Medium leichter in Schwingungen versetzen, als es bei einem prall gefüllten Beutel der Fall ist.

Entsprechend einer vorteilhaften Ausführungsform der Erfindung wirkt das Druckglied auf einen Randbereich des oder der Kunststoffbeutel ein. Dies hat nicht nur den Vorteil, daß der mittlere Bereich der Kunststoffbeutel als Berührungsfläche für das medizinische Produkt zur Verfügung steht, sondern von einem Rand der Kunststoffbeutel her lassen sich im übrigen auch die angestrebten Schwingungen zuverlässiger erzielen. Im Bereich der Einwirkung des Druckgliedes können die Kunststoffbeutel eine Verstärkung aufweisen, die den Verschleiß verringert und im übrigen auch flächig ausgebildet sein kann, so daß sie den Druck des Druckgliedes über eine größere Fläche der Kunststoffbeutel verteilt.

Im folgenden werden bevorzugte Ausführungsbeispiele der Erfindung anhand der beigefügten Zeichnung näher erläutert.

Fig. 1    ist eine perspektivische, teilweise aufgebrochene Gesamtdarstellung einer erfindungsgemäßen Vorrichtung;

Fig. 2    ist ein schematischer Längsschnitt durch die Vorrichtung.

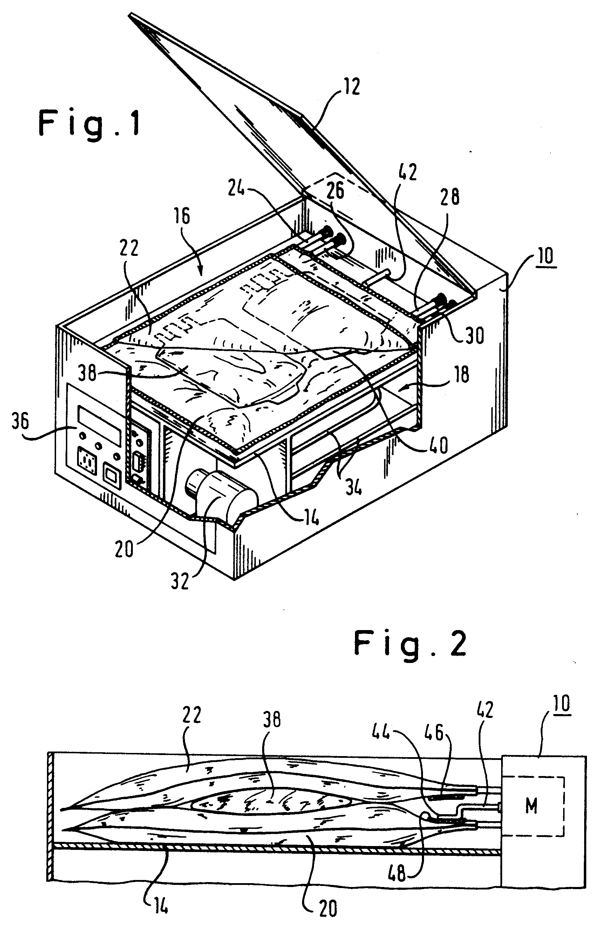

Fig. 1 zeigt eine erfindungsgemäße Vorrichtung mit einem kastenförmigen Gehäuse 10, an dessen Oberseite ein aufklappbarer Deckel 12 vorgesehen ist. Das Innere des Gehäuses ist durch eine waagerechte Platte 14 in eine obere Temperierkammer 16 und eine untere Warmwasserkammer 18 unterteilt.

In der Temperierkammer 16 liegt auf der Platte 14 ein rechteckiger Kunststoffbeutel 20 und auf diesem ein im wesentlichen deckungsgleicher, in Fig. 1 zurückgeklappt gezeigter Kunststoffbeutel 22. Die Kunststoffbeutel bestehen beispielsweise aus einem weichen, temperaturbeständigen Material wie z.B. PVC. Beide Kunststoffbeutel weisen Schlauchanschlüsse 24,26,28,30 auf, die jeweils paarweise Ein- und Auslaß der beiden Kunststoffbeutel 20,22 bilden. Die Schlauchanschlüsse 24,26,28,30 sind in nicht gezeigter Weise mit einer Pumpe 32 verbunden, die warmes Wasser aus der Warmwasserkammer 18 durch die Kunststoffbeutel 20, 22 umwälzt. Dabei sind die Schlauchanschlüsse der Kunststoffbeutel gegenläufig geschaltet, so daß beispielsweise die Schlauchanschlüsse 24,26, die links in Fig. 1 gezeigt sind, für den unteren Kunststoffbeutel den Einlaß und für den oberen Kunststoffbeutel den Auslaß bilden. Dadurch wird die Wirkung eines in den Kunststoffbeuteln auftretenden Temperaturabfalls ausgeglichen.

In der Warmwasserkammer 18, in der sich trotz dieser Bezeichnung auch ein anderes Temperiermedium befinden kann, sind Heizschlangen 34 vorgesehen, die in Verbindung mit einer an der Stirnseite des Gehäuses 10 angeordneten Steuerelektronik 36 eine vollständig gleichmäßige und genaue Temperatureinstellung des Temperiermediums ermöglichen.

Bei Anwendung der erfindungsgemäßen Vorrichtung etwa zur Erwärmung von Plasmabeuteln 38,40 wird der obere Kunststoffbeutel 22 hochgehoben, die Plasmabeutel werden auf den unteren Kunststoffbeutel 20 aufgelegt, und der obere Kunststoffbeutel 22 wird wiederum auf die Plasmabeutel aufgelegt. Aufgrund des weichen Materials der Kunststoffbeutel schließen diese sich eng und flächig an die Plasmabeutel an, so daß eine rasche Wärmeübertragung stattfinden kann.

Während des Temperiervorganges bleiben die Plasmabeutel vollständig trocken. Da das warme Wasser innerhalb eines geschlossenen Systems verbleibt und mit dem medizinischen Produkt nicht in Berührung kommt, ist es nicht erforderlich, nach jeder Benutzung das Wassersystem zu reinigen und zu sterilisieren. Für den Fall, daß die Kunststoffbeutel 20,22 ausnahmsweise Leckstellen aufweisen sollten, ist die Temperierkammer 16 zusammen mit der Platte 14 vorzugsweise als wasserdichte Auffangwanne ausgebildet, in der Flüssigkeitsspuren rasch bemerkt werden können, so daß deren Ursache beseitigt werden kann.

Entgegen der in Fig. 1 gezeigten Ausführungsform kann der obere Kunststoffbeutel 22 an dem Deckel 12 aufgehängt sein, so daß er beim Öffnen des Deckels automatisch angehoben wird.

Aus der rückwärtigen Wand der Temperierkammer 16, bezogen auf Fig. 1, tritt zwischen den Schlauchanschlüssen 24,26 sowie 28,30 eine Welle 42 aus, die außerhalb der Temperierkammer 16 mit einem nicht gezeigten Antriebsmotor verbunden ist. Die Welle 42 erstreckt sich in eine Position zwischen den Kunststoffbeuteln 20,22, wie in Fig. 2 näher gezeigt ist. Zwischen den Kunststoffbeuteln 20,22 trägt die Welle 42 an ihrem Ende eine Kurbel oder einen sonstigen Exzenter 44, der bei Drehung der Welle 42 eine Kreisbewegung beschreibt und dabei abwechselnd den unteren und den oberen Kunststoffbeutel 20,22 in dessen Randbereich zusammendrückt. Durch dieses Zusammendrücken wird das Temperiermedium innerhalb der Beutel 20,22 nach links in Fig. 2 verschoben, so daß das Temperiermedium in den gesamten Kunststoffbeuteln in Schwingungen gerät, die sich dem Plasmabeutel 38 mitteilen. Dadurch gerät auch das Plasma in Schwingung, so daß sich der erwähnte Vorteil einer besonders raschen Temperaturverteilung innerhalb des Plasma ergibt.

An den von dem Exzenter 44 beaufschlagten Rändern der Kunststoffbeutel 20,22 können Verstärkungen 46,48 vorgesehen sein, die den Verschleiß bei Einwirkung des Exzenters 44 verringern. Der Exzenter 44 kann im übrigen auch einen verschleißhindernden Überzug, etwa aus Polytetrafluoräthylen aufweisen. Die Verstärkungen 46,48 können streifenförmig oder in anderer Form über Teilflächen der Kunststoffbeutel 20,22 hinweg verlaufen und aus relativ steifem Material bestehen, so daß sich der ausgeübte Druck auf eine größere Fläche der Kunststoffbeutel verteilt.

**Patentansprüche**

1.  Auftau- und Temperiervorrichtung für medizinische Produkte, insbesondere für tiefgefrorene Plasma- oder Blutkonserven (38), mit wenigstens zwei ein temperiertes Medium enthaltenden, beidseitig eines zu temperierenden Produkts anzuordnenden Kunststoffbeuteln (20, 22), **gekennzeichnet** durch wenigstens ein periodisch auf wenigstens einen der Kunststoffbeutel (20,22) einwirkendes Druckglied (42,44).

2.  Auftau- und Temperiervorrichtung nach Anspruch 1, dadurch **gekennzeichnet,** daß zwei aufeinander liegende, im wesentlichen deckungsgleiche, mit dem temperierten Medium gefüllte Kunststoffbeutel vorgesehen sind und das Druckglied einen zwischen die Kunststoffbeutel (20,22) eintretenden, umlaufenden, alternativ auf einen der Kunststoffbeutel (20,22) einwirkenden Exzenter (44) umfaßt.

3.  Auftau- und Temperiervorrichtung nach Anspruch 1 oder 2, dadurch **gekennzeichnet,** daß durch die Kunststoffbeutel ein flüssiges, temperiertes Medium mit Hilfe einer Pumpe (32) umgewälzt wird und der Innendruck bzw. Füllungsgrad der Kunststoffbeutel (20,22) veränderbar ist.

4. Auftau- und Temperiervorrichtung nach Anspruch 3, dadurch **gekennzeichnet,** daß der Pumpenausgangsdruck veränderbar ist.

5. Auftau- und Temperiervorrichtung nach einem der vorhergehenden Ansprüche, dadurch **gekennzeichnet,** daß das Druckglied (42,44) auf Randbereiche der Kunststoffbeutel (20,22) einwirkt.

6. Auftau- und Temperiervorrichtung nach einem der vorhergehenden Ansprüche, dadurch **gekennzeichnet,** daß die Kunststoffbeutel (20,22) im Bereich der Einwirkung das Druckgliedes (42,44) eine Verstärkung (46,48) aufweisen.

7. Auftau- und Temperiervorrichtung nach Anspruch 6, dadurch **gekennzeichnet,** daß die Verstärkung (46,48) aus verhältnismäßig steifem Material besteht und sich streifenförmig über die Kunststoffbeutel (20,22) erstreckt.

8. Auftau- und Temperiervorrichtung nach einem der vorhergehenden Ansprüche 3 bis 7, dadurch **gekennzeichnet,** daß eine Steuervorrichtung zur selbsttätigen Steuerung eines Temperiervorganges, vorgesehen ist und die Tätigkeit des Druckgliedes (42,44) steuerungstechnisch gekoppelt ist mit einer Reduzierung des Pumpenausgangsdrucks der Pumpe (32).

## Claims

1. Thawing and tempering apparatus for medical products, particularly for deep frozen plasma and blood conserves (38), comprising at least two plastic bags (20,22) containing a tempered medium to be positioned on both sides of a product to be tempered, **characterized** by at least one pressure member (42,44) periodically acting upon at least one of the plastic bags (20,22).

2. Thawing and tempering apparatus according to claim 1, **characterized** in that two substantially congruent plastic bags lying one on top of the other and filled with the tempered medium are provided, and that the pressure member comprises an eccentric, rotatable cam (44) entering between the plastic bags (20,22) alternatively acting upon one of the plastic bags (20,22).

3. Thawing and tempering apparatus as claimed in claim 1 or 2, **characterized** in that a liquid, tempered medium is circulated through the plastic bags by means of a pump (32), and that the interior pressure or filling ratio of the plastic bags (20,22) is variable.

4. Thawing and tempering apparatus as claimed in claim 3, **characterized** in that the pump output pressure is variable.

5. Thawing and tempering apparatus according to one of the preceding claims, **characterized** in that the pressure member (42,44) acts upon edge portions of the plastic bags (20,22).

6. Thawing and tempering apparatus according to one of the preceding claims, **characterized** in that the plastic bags (20,22) are provided with a reinforcement (46,48) in the portions acted upon by the pressure member (42,44).

7. Thawing and tempering apparatus according to claim 6, **characterized** in that the reinforcement (46,48) consists of comparatively stiff material and extends striplike over the plastic bags (20,22).

8. Thawing and tempering apparatus according to one of the preceding claims 3 to 7, **characterized** in that a control unit is provided for automatic control of the tempering process and that the operation of the pressure member (42,44) is controlled in coordination to a reduction of the pump output pressure of the pump (32).

## Revendications

1. Dispositif de décongélation et de réchauffage pour produits médicaux, en particulier pour des conserves de plasma ou de sang congelé (38), équipé d'au moins deux sacs de matière plastique (20,22) contenant un fluide réchauffé, disposés de part et d'autre d'un produit à réchauffer, caractérisé par au moins un élément de pression (42,44) agissant périodiquement sur au moins un des sacs de matière plastique (20,22).

2. Dispositif de décongélation et de réchauffage selon la revendication 1, caractérisé en ce que deux sacs de matière plastique superposés, sensiblement de même étendue, remplis du fluide réchauffé, sont prévus et l'élément de pression comporte un excentrique (44) tournant pénétrant entre les sacs de matière plastique (20,22), et agissant alternativement sur l'un des sacs de matière plastique (20,22).

3. Dispositif de décongélation et de réchauffage selon la revendication 1 ou 2, caractérisé en ce qu'à travers les sacs de matière plastique circule un fluide liquide, réchauffé, à l'aide d'une pompe (32), et la pression intérieure ou le degré de remplissage des sacs de matière plastique (20,22) est réglable.

4. Dispositif de décongélation et de réchauffage selon la revendication 3, caractérisé en ce que la pression de sortie de la pompe est réglable.

5. Dispositif de décongélation et de réchauffage selon l'une des revendications précédentes, caractérisé en ce que l'élément de pression (42,44) agit sur des zones de bord des sacs de matière plastique (20,22).

6. Dispositif de décongélation et de réchauffage selon l'une des revendications précédentes, caractérisé en ce que les sacs de matière plastique (20,22) présentent un renfort (46,48) dans la region d'action de l'élément de pression (42,44).

7. Dispositif de décongélation et de réchauffage selon la revendication 6, caractérisé en ce que le renfort (46,48) est en un matériau relativement rigide et s'étend sous forme de bande sur les sacs de matière plastique (20,22).

8. Dispositif de décongélation et de réchauffage selon l'une des revendications 3 à 7, caractérisé en ce qu'un dispositif de commande en vue de la commande automatique d'un processus de réchauffage est prévu et en ce que l'action de l'élément de pression (42,44) est couplée de façon commandée à une réduction de la pression de sortie de la pompe (32).

Fig.1

Fig.2